# EUROPÄISCHE PATENTSCHRIFT

(11) **EP 1 309 729 B1**
(45) Veröffentlichungstag und Bekanntmachung des Hinweises auf die Patenterteilung: **22.12.2004**
(21) Anmeldenummer: 01974091.9
(22) Anmeldetag: 26.07.2001
(51) Int. Cl.: C12Q 1/68, G01N 1/31, G01N 33/543

(54) **VERWENDUNG EINES BILDGEBENDEN PHOTOELEKTRISCHEN FLÄCHENSENSORS ZUR AUSWERTUNG VON BIOCHIPS UND BILDGEBUNGSVERFAHREN HIERFÜR**
USE OF AN IMAGING PHOTOELECTRIC FLAT SENSOR FOR EVALUATING BIOCHIPS AND IMAGING METHOD THEREFOR
UTILISATION D'UN DETECTEUR PLAT PHOTOELECTRIQUE D'IMAGERIE POUR L'ANALYSE DE BIOPUCES, ET PROCEDE D'IMAGERIE CONNEXE

(30) Priorität: 26.07.2000 DE 10036457
(43) Veröffentlichungstag der Anmeldung: 14.05.2003
(73) Patentinhaber: Giesing, Michael, 45659 Recklinghausen (DE)
(72) Erfinder: GIESING, Michael, 45659 Recklinghausen (DE); GRILL, Hans-Jörg, 45659 Recklinghausen (DE); LECLERC, Norbert, 07743 Jena (DE); SCHÜTZ, Andreas, 45739 Oer-Erkenschwiek (DE); PRIX, Lothar, 45663 Recklinghausen (DE)
(74) Vertreter: Müller, Jörg Uwe
(86) Internationale Anmeldenummer: PCT/EP2001/008673
(87) Internationale Veröffentlichungsnummer: WO 2002/008458

(56) Entgegenhaltungen:
- WO-A-93/22678
- WO-A-95/23348
- WO-A-98/29736
- WO-A-99/27140
- US-A- 5 677 196
- US-A- 6 078 705
- PATENT ABSTRACTS OF JAPAN vol. 012, no. 399 (P-775), 24. Oktober 1988 (1988-10-24) & JP 63 139235 A (SHISEIDO CO LTD;OTHERS: 01), 11. Juni 1988 (1988-06-11)

## Beschreibung

Die Erfindung betrifft die Verwendung eines bildgebenden photoelektrischen Flächensensors zur Auswertung von Biochips und ein Bildgebungsverfahren hierfür. Die Erfindung betrifft insbesondere ein Verfahren zur orisaufgeiösten Detektion von elektromagnetischer Strahlung, die von auf einer Oberfläche eines planaren Trägers immobilisierten Substanzen emittiert wird, mittels eines bildgebenden photoelektrischen Flächensensors.

Das Erkennen bestimmter genetischer Informationen ist eine elementare molekularbiologische Aufgabenstellung, zu deren Lösung bereits eine große Zahl unterschiedlichster Methoden vorgeschlagen wurde. Kann eine gesuchte genetische Information auf bestimmte Nukleinsäuresequenzen (im folgenden als Targetsequenzen oder Targets bezeichnet) zurückgeführt werden, benutzt man in vielen Fällen sogenannte Oligonukleotidsonden, deren Nukleinsäuresequenzen zu den Targetsequenzen komplementär sind. Aufgrund ihrer Komplementarität können die Oligonukleotidsonden und die Targetsequenzen spezifisch hybridisieren, so daß man die gesuchten Targetsequenzen in einem Pool umfangreicher und komplexer genetischer Informationen identifizieren und qualitativ und/oder quantitativ analysieren kann.

Klassische Anwendungen dieser Art sind Northem- und Southem-Blots, sowie die *in-situ* Hybridisierung. Dazu werden die Proben in der Regel zweckgerecht vorbereitet und mit Hilfe definierter Oligonukleotidsonden untersucht. Bei herkömmlichen Anwendungen dieser Art sind üblicherweise die 0ligonukleotidsonden markiert und können so, abhängig von der gewählten Markierung, detektiert werden. Dies ist erforderlich, um probengebundene Sonden, d.h. Sonden, die spezifisch an Targetsequenzen hybridisiert haben, erkennen zu können.

Zur Markierung der Sonden werden Substanzen, sog. Marker, eingesetzt, die mit Hilfe geeigneter Nachweismethoden identifiziert werden können. Weit verbreitet sind insbesondere radioaktive Marker, sowie Chemilumineszenz- oder Fluoreszenzmarker.

Dabei haben insbesondere Fluoreszenz- und Chemilumineszenzmethoden in der chemischen und biologischen Analytik und Diagnostik einen hohen Stellenwert. Es handelt sich um sehr nachweisstarke Methoden, die ohne Radioaktivität und, falls erforderlich, ohne toxische Substanzen auskommen. Viele der verwendeten Marker sind verglichen mit Radioisotopen bei entsprechender Lagerung praktisch unbegrenzt haltbar. Es existieren heute empfindliche optische Detektionssysteme, die schon den Nachweis einzelner Markermoleküle ermöglichen. Außerdem existiert eine Vielfalt unterschiedlichster Fluoreszenzfarbstoffe, so daß für die meisten Wellenlängenbereiche im sichtbaren Spektrum, aber auch im angrenzenden ultravioletten und infraroten Spektralbereich auf geeignete Fluoreszenzmarker zurückgegriffen werden kann. Entsprechend sind geeignete Chemilumineszenzsubstrate für viele Enzyme, beispielsweise Peroxidasen, Alkalische Phosphatase, Glucose-Oxidase und andere verfügbar. Leistungsfähige Chemilumineszenzsubstrate mit einer Signalstabilität von über einer Stunde sind kommerziell erhältlich.

Bei den oben erwähnten klassischen Hybridisierungsmethoden ist die Zahl der unterschiedlichen Sonden, die in Verbindung mit ein und derselben Probe eingesetzt werden können, begrenzt. Um die Sonden unterscheiden zu können, sind unterschiedliche, d.h. nicht interferierende Markierungen und in der Folge auch unterschiedliche Detektionssysteme erforderlich. Der damit verbundene Aufwand stößt spätestens bei Multi-Parameter-Analysen an die Grenzen der Praktikabilität.

Entscheidende Vorteile bieten in dieser Hinsicht Testanordnungen mit immobilisierten Oligonukleotidsonden, d.h. Sonden, die an einen festen Träger fixiert sind. Um bei derartigen Systemen eine Bindung von Probe und Sonde erkennen zu können, wird in diesen Fällen die Probe und nicht die Sonde markiert. Unter einem festen Träger versteht man dabei ein Material mit starrer oder halbstarrer Oberfläche. Bei derartigen Trägem kann es sich beispielsweise um Partikel, Stränge, insbesondere Faserbündel, sphärische Körper, wie Kugeln oder Kügelchen (sogenannte "beads"), Fällungsprodukte, Gele, Blätter, Röhren, Behältnisse, Kapillarröhrchen, Scheiben, Folien oder Platten handeln. Am weitesten verbreitet sind inzwischen jedoch planare, d.h. ebene Träger.

Soll eine Probe mittels mehrerer Sonden mit unterschiedlicher Spezifität untersucht werden, so werden diese Sonden üblicherweise auf einem gemeinsamen Träger so angeordnet, daß jede Sondenart, also beispielsweise eine bestimmte Oligonukleotidsonde mit bekannter Sequenz, einem bestimmten Feld eines zweidimensionalen Feldmusters (allgemein als "Array" bezeichnet) auf dem Träger zugeordnet wird. Die Feststellung, ob und/oder gegebenenfalls in welchem Maße die markierte Probe an ein bestimmtes Feld bindet, erlaubt Rückschlüsse auf die zur Sonde dieses Feldes komplementäre Targetsequenz der Probe und gegebenenfalls auf deren Konzentration.

Durch fortschreitende Miniaturisierung konnten die Felder mittlerweile wesentlich verkleinert werden, so daß man heute eine Vielzahl verfahrens- und meßtechnisch unterscheidbare Felder, also auch eine Vielzahl unterscheidbarer Sonden auf einem einzelnen Träger anordnen kann. Obwohl Glasträger im molekularbiologischen Bereich für diese Zwecke noch am weitesten verbreitet sind, werden die planaren Träger in Anlehnung an die Halbleitertechnologie auch als "Chips", insbesondere als Biochips, Genchips usw. bezeichnet. Die Sonden können in sehr hoher Dichte an den Träger gebunden und mehrere Sonden einer Sondenart in einem miniaturisierten Feld angeordnet werden. Heute können bereits Chips mit bis zu 40.000 unterschiedlichen molekularen Sonden pro cm² hergestellt werden.

Vor allem hat die Anwendung photolithographischer Fabrikationstechniken aus der Halbleitertechnologie zu entscheidenden Fortschritten bei der Herstellung solcher Chips geführt. Das Prinzip basiert auf einer lichtgesteuerten chemischen Festphasensynthese, bei der photolithographische Masken die Felder abbilden (vgl. beispielsweise Fodor et al, "Lightdirected, spatially addressable parallel chemical synthesis", Science, vol. 251, 767-773 (1991)). Dieses Verfahren ist insbesondere dann von Vorteil, wenn die Sonden auf dem Träger *in-situ* aus einzelnen Bausteinen, beispielsweise Nukleotiden, aufgebaut werden sollen. So kann ein bestimmter Baustein gezielt an die im Aufbau befindlichen Sonden bestimmter Felder angefügt werden, während die Sonden der übrigen Felder unberührt bleiben. Dies gelingt mit photolithographischen Masken, die Licht zur lichtgesteuerten chemischen Synthese nur auf diejenigen Felder abbilden, an die der Baustein angefügt werden soll. Beispielsweise kann der Lichteinfall die Abspaltung lichtempfindlicher Schutzgruppen bewirken, wodurch eine reaktive Gruppe genau an derjenigen Stelle der im Aufbau befindlichen Sonden freigesetzt wird, an die der Baustein angefügt werden soll. Da ein zuletzt angefügter Baustein in der Regel eine gebundene Schutzgruppe einbringt und dadurch die um einen Baustein erweiterten Sonden wieder schützt, wird an eine aktivierte Sonde lediglich ein einziger Baustein angefügt. Aus dem gleichen Grund stehen die während eines Zyklus um einen Baustein erweiterten Sonden gleichermaßen wie die in diesem Zyklus nicht erweiterten Sonden als zunächst geschützte Gesamtheit aller Sonden einer neuen gezielten Aktivierung durch eine passende Maske für die Anfügung eines weiteren Baustein in einem neuen Zyklus zur Verfügung. Derartige Verfahren werden ausführlich in den internationalen Patentanmeldungen WO 90/15070, WO 91/07087, WO 92/10092, WO 92/10587, WO 92/10588 und in dem U.S.-Patent 5,143,854 beschrieben.

Andere Chips weisen wiederum Sonden auf, die nicht *in-situ* synthetisiert, sondern in vorgefertigter Form auf den Träger aufgebracht werden. Entsprechende Arrays aus Biomolekülen zur Sequenzanalyse von Polynukleotiden wurden bereits von E. Southem in der internationalen Patentanmeldung WO 89/10977 beschrieben. Biomotekulare Arrays eignen sich für eine Vielzahl von Anwendungen, angefangen von der Sequenzierung von DNA über das DNA-Fingerprinting bis zu Anwendungen in der medizinischen Diagnostik. Inzwischen werden schon kommerzielle Biochips mit einer Vielzahl verschiedener cDNAs zur Hybridisierung angeboten. Bei diesen cDNAs handelt es sich beispielsweise um Nukleinsäuresequenzen mit Längen von etwa 200 bis 600 Basenpaaren (bp), die mittels genspezifischer Primer amplifiziert werden, deren Identität durch Teilsequenzierung überprüft wird und die dann gezielt an bekannten Plätzen beispielsweise auf einer Nylonmembran angebracht werden.
Werden die markierten Proben mit dem planaren Chip in Kontakt gebracht, so kann es auf einzelnen Feldern zu einer Kopplung, beispielsweise einer Hybridisierung, mit komplementären Sonden kommen. In Fällen, in denen eine Markierung der Proben nicht zweckmäßig ist, kann auch nach Bindung der Proben an die Sonden der Chip mit geeigneten markierten Rezeptoren, die spezifisch an die Proben binden, in Kontakt gebracht werden. In beiden Fällen werden Fluoreszenz- oder Chemilumineszenzmarker auf denjenigen Feldelementen immobilisiert, wo eine Bindung zwischen Sonden und Proben stattgefunden hat. Bei fluoreszenzoptischen Nachweismethoden wird der Chip dann mit Licht einer geeigneten Wellenlänge bestrahlt, so daß die Fluoreszenzfarbstoffe angeregt werden und Fluoreszenzstrahlung emittieren. Bei einer Nachweismethode durch Lumineszenz wird kein externes Anregungslicht benötigt. Vielmehr verwendet man chemilumineszierenden oder biolumineszierenden Systeme als Marker zur Signalerzeugung. Die Fluoreszenz- oder Lumineszenzstrahlung erzeugt ein Muster aus hellen und dunklen Feldelementen auf dem planaren Träger, das aufgezeichnet wird. Informationen über die Probe kann man also dadurch erhalten, daß man das Hell/Dunkel-Muster mit dem bekannten Muster der an die Trägeroberfläche fixierten biologischen Sonden vergleicht.

Die fortschreitende Miniaturisierung hat zu einer großen Anzahl von Feldelementen auf einem einzelnen planaren Träger geführt, die bei kommerziellen Anwendungen in kurzer Zeit sehr zuverlässig vermessen werden müssen. Zum ortsaufgelösten fluoreszenzoptischen Nachweis von auf einem Biochip immobilisierten Substanzen werden heute hauptsächlich sogenannte Scanner eingesetzt, bei denen die Oberfläche des Chips mit einem fokussierten Laserstrahl abgetastet und das emittierte Fluoreszenzlicht detektiert wird. Ein entsprechender Fluoreszenzscanner wird von der Firma Hewlett-Packard für die Auswertung von Biochips der Firma Affymetrix hergestellt und ist in den US-Patenten 5,837,475 und 5,945,679 detaillierter beschrieben. Es sind auch Scanner bekannt, bei denen ein konfokales Anregungs- und Detektionssystem in ein Epifluoreszenzmikroskop integriert ist. Als Detektionssysteme für das emittierte Fluoreszenzlicht werden bei Scannern meist sogenannte Einkanalsysteme, also beispielsweise einzelne Photozellen oder Sekundärelektronenvervielfacher (Photomultiplier) verwendet.

Es sind jedoch auch zweidimensionale Detektionssysteme, wie beispielsweise CCD-Kameras bekannt, die sowohl zur Detektion von Fluoreszenzlicht als auch zur Detektion von Chemilumineszenzlicht einer Probe verwendet werden können. Kommerziell erhältliche Systeme besitzen entweder ein optisches Abbildungssystem, welches unter Verwendung von Linsenoptiken die mit Chemilumineszenzmarkem oder Fluoreszenzmarkem versehene Biochipoberfläche auf einen CCD-Sensor abbilden, oder eine Kombination aus einem Bildverstärker (Image Intensifier) und einer CCD-Kamera.

So wird in DE 197 36 641 A1 ein optisches Meßsystem für die Biosensorik beschrieben, welches beispielsweise einen CCD-Chip als Detektor verwendet. Das Messobjekt und der CCD-Chip sind durch optische Einrichtungen verbunden, die Fasern, Linsen und Spiegel umfassen können.

In US 5,545,531 werden zahlreiche Detektionssysteme zur Untersuchung von sogenannten Biochip-Assays beschrieben, u.a. Scannersysteme und CCD-Systeme mit lichtstarker Abbildungsoptik. Erwähnt wird auch die Möglichkeit eines Einbaus eines CCD-Arrays in den Waver einer "biochip plate", ohne dem Fachmann aber diesbezüglich eine klare und nachvollziehbare technische Lehre zu offenbaren.

In US 5,508,200 wird die Auswertung chemischer Assays mittels einer Videokamera beschrieben, die mit einer Abbildungsoptik versehen ist

In der Internationalen Patentanmeldung WO 97/35181 (PCT/US97/04377) wird ein Immunoassay-System beschrieben, bei welchem fluoreszenzmarkierte Moleküle eines Biosensors durch evaneszentes Licht angeregt und die von diesen Molekülen emittierte Fluoreszenzstrahlung von einem Photodetektorarray registriert wird. Um Fluoreszenz aus unterschiedlichen Bereichen des Biosensors optisch voneinander zu trennen, wird die Fluoreszenzstrahlung wird durch sogenannte "Tunnel" mit lichtundurchlässigen Wänden von dem Biosensor zu den den jeweiligen Bereichen zugeordneten Photodetektoren geleitet.

Die bekannten Fluoreszenz- oder Lumineszenzdetektionssysteme für Biochips weisen jedoch Nachteile auf. So sind CCD-Kameras mit einer Linsenoptik meist recht teuer, da entweder aufwendig korrigierte asphärische Objektive verwendet werden oder bei Einsatz einer weniger aufwendigen Abbildungsoptik eine Korrektur der Bildverzerrung und Vignettierung durch eine komplexe Bildbearbeitungssoftware erforderlich ist. Für eine ausreichend hohe Empfindlichkeit ist es außerdem meist notwendig, mit gekühlten CCD-Sensoren oder sogenannten "Slow-Scan, Full-Frame Scientific Chips" zu arbeiten. Auch die Verwendung von Bildverstärkem ist neben den hohen Kosten mit weiteren Nachteilen verbunden. So ist zum Betrieb eines Bildverstärkers ein Hochspannungsanschluß an der Abbildungsoptik erforderlich, was beim Arbeiten mit wäßrigen Medien, wie beispielsweise Puffern, Gefahren für den Benutzer mit sich bringt. Soweit Faseroptiken verwendet werden, treten Verluste beim Einund Auskoppeln des Fluoreszenz- oder Lumineszenzlichtes in die Fasern auf. Außerdem ist die Auflösung von faseroptischen Abbildungssystemen begrenzt. Das aus "Lichttunneln" bestehende Abbildungssystem der oben erwähnten WO 97/35181 weist zudem den Nachteil auf, dass nur Lichtstrahlen, die im wesentlichen parallel zur Tunnelachse verlaufen, den Detektor erreichen können. Außerdem müssen die einzelnen Felder des Biosensors, die "Lichttunnel" und die Detektoren exakt zueinander ausgerichtet werden.

Der vorliegenden Erfindung liegt daher das technische Problem zugrunde, ein einfaches und kostengünstiges Bildgebungssystem zur ortsaufgelösten Detektion von elektromagnetischer Strahlung, insbesondere Lumineszenz- und/oder Fluoreszenzstrahlung, die von auf einer planaren Oberfläche eines Trägers, insbesondere eines Biochips, immobilisierten Substanzen emittiert wird, anzugeben. Das Bildgebungssystem soll dabei einfach zu handhaben sein.

Zur Lösung dieses Problems schlägt die Erfindung vor, einen bildgebenden photoelektrischen Flächensensor zur Kontaktabbildung einer Oberfläche eines Biochips zu verwenden. Überraschenderweise wurde gefunden, daß es möglich ist, die Fluoreszenz- oder Lumineszenzstrahlung, die von auf einer im wesentlichen planaren Oberfläche eines Biochips immobilisierten Substanzen emittiert wird, ortsaufgelöst und mit hoher Empfindlichkeit zu detektieren, indem man einen bildgebenden photoelektrischen Flächensensor in möglichst geringem Abstand von der Oberfläche des Biochips anordnet, die immobilisierten Substanzen zur Emission von elektromagnetischer Strahlung, vorzugsweise von Licht im sichtbaren und/oder infraroten und/oder ultravioletten Spektralbereich anregt, und die emittierte Strahlung ohne Verwendung eines abbildenden optischen Systems photoelektrisch detektiert. Unter einem abbildenden optischen System" ist im vorliegenden Zusammenhang jegliche Einrichtung zu verstehen, welche elektromagnetische Strahlung, die von einem Bereich der Oberfläche des Biochips ausgeht in eindeutiger Weise einem bestimmten Bereich des Flächensensors zuordnet, also insbesondere Linsen- und Spiegelsysteme, Gradientenlinsenarrays, Faser- oder Lichtwellenfeiterbündel, aber auch eine Anordnung mehrer "Lichttunnel" wie sie in WO 97/35181 beschrieben sind. Vielmehr ist im Sinne der vorliegenden Erfindung zwischen der Oberfläche des Biochips und der Oberfläche des Flächensensors allenfalls eine dünne transparente Platte (beispielsweise ein Glasplättchen) und/oder ein dünner Fluidspalt (beispielsweise ein Luft- oder Flüssigkeitsspalt) vorgesehen. Überraschend wurde nämlich gefunden, dass es bei hinreichend geringem Abstand zwischen Biochip und Flächensensor jedem photoelektrische Element des Flächensensors ein definierter Bereich des Biosensors zugeordnet ist und das entsprechende Element nicht gegen Streulicht von benachbarten Bereichen des Flächensensors abgeschirmt werden muss.

Unter dem Begriff "Anregung" ist hier nicht nur eine Anregung durch Einstrahlung von elektromagnetischer Strahlung zu verstehen, wie dies beispielsweise zur Fluoreszenzdetektion erforderlich ist. Vielmehr soll der Begriff "Anregung" jegliche Beeinflussung der immobilisierten Substanzen umfassen, die mit der anschließenden Emission von Licht verbunden ist, insbesondere auf der Basis von Chemi- oder Biolumineszenz. Wenn hier von "immobilisierten Substanzen" die Rede ist, so bedeutet dies nicht, daß die entsprechenden Substanzen völlig unbeweglich sind. Vielmehr soll damit ausgedrückt werden, daß die Beweglichkeit der Sonden innerhalb eines Inkubations- und/oder Meßintervalls, also im Sekunden- oder Minutenbereich, so gering ist, daß noch eine eindeutige räumliche Zuordnung der Substanz zu einem Feldelement des Biochips möglich ist

Mit der Erfindung sind zahlreiche Vorteile verbunden:
Das erfindungsgemäße Detektionssystem ist besonders kostengünstig, da es ohne Linsenoptiken, Bildverstärker oder Faseroptiken zur Abbildung des Biochips auf dem Flächensensor auskommt. Herkömmliche, mit Optiken arbeitende Systeme müssen hohe Signalverluste durch die Optik in Kauf nehmen. Außerdem kommt durch den kleineren Raumwinkel der optischen Abbildungssysteme über eine größere Distanz nur ein Bruchteil des Ausgangssignals am Detektor an. Diese Verluste müssen bei den bekannten Detektoren durch teure Hochleistungsdetektoren oder elektronische Bildverstärker ausgeglichen werden. Dagegen ist es bei der vorliegenden Erfindung durch einen Verzicht auf jegliche Abbildungsoptik und durch die unmittelbare räumliche Nähe von Signalerzeugung und Detektion möglich, emittiertes Licht aus einem großen Raumwinkelbereich zu empfangen. Daher kann auf einfachere und kostengünstige Detektoren zurückgegriffen werden kann. Die hohe Signalausbeute ermöglicht außerdem sehr kurze Meßzeiten - in manchen Fällen genügt eine Meßzeit von weniger als 50 ms für einen kompletten Biochip.
Das Detektionssystem ist gegenüber dem Stand der Technik kompakter, hochgradig miniaturisiert und einfach zu bedienen, da keine Fokussierung oder Justierung vorgenommen werden muß. Durch Verzicht auf ein optisches Abbildungssystem können auch keine Bildverfälschungen, wie Vignettierung, Verzerrung oder Änderung der Dynamik auftreten. Aufgrund seiner Kompaktheit und seiner Miniaturisierung ist das erfindungsgemäße Detektionssystem leicht in automatische Analysensysteme integrierbar.
Bei ähnlicher Empfindlichkeit ist die Bedienung wesentlich einfacher und schneller als bei einem Röntgenfilm. Außerdem erhält man direkt digitalisierte Daten, die weiterverarbeitet werden können.
Eine komplizierte Ausrichtung und Justierung des Biochips zu dem Flächensensor ist nicht erforderlich, da das Auffinden und die Identifizierung des einzelnen Spots auch nach einer Datenerfassung auf softwaretechnischem Weg erfolgen kann.

Als Flächensensor werden bevorzugt ein Dioden- oder Transistorarray, ein CCD-Sensor (etwa ein Video-, Full Frame- oder Zeilensensor, ein Slow-Scan Scientific CCD oder auch ein Line-Transfer-Model) oder ein TFA-Bildsensor verwendet. TFA steht dabei für "Thin Film on ASIC (Application Specific Integrated Circuit)". TFA-Bildsensoren bestehen beispielsweise aus einer dünnen Schicht aus amorphem Silizium auf einem ASIC-Sensor. Auch Zeilenarrays sollen in diesem Zusammenhang unter den Begriff "Flächensensor" fallen, da beispielsweise lineare Zeilenarrays aufgrund ihrer endlichen Querabmessungen stets eine bestimmte Fläche des Biochips bedecken.

Das Detektionssystem besteht demnach gemäß einer bevorzugten Ausführungsform aus einem bildgebenden Flächensensor und einem Biochip, der für die Messung direkt auf die Sensorfläche gelegt wird. Zwischen Flächensensor und Biochip kann ein Abstandshalter angeordnet sein, der beispielsweise einen Reaktionsraum definiert, der im Fall einer Chemioder Biolumineszenznachweismethode mit Lumineszenzsystemkomponenten, in der Regel an der Lumineszenzreaktion beteiligten Reaktanden, z.B. Chemilumineszenzsubstrat, oder, falls das Substrat auf der Chipoberfläche fixiert ist, mit Enzymlösung gefüllt werden kann. Durch Aktivierung des Substrats wird Chemilumineszenz- oder Biolumineszenzstrahlung emittiert und vom Flächensensor ortsaufgelöst photoelektrisch detektiert.

Typischerweise werden Flächensensoren mit mehr als 10.000 Pixeln verwendet. Die Sensorfläche ist vorzugsweise wenigstens so groß wird die abzubildende Oberfläche des Biochips und beträgt üblicherweise 40 bis 100 mm². Da sämtliche Pixel des Flächensensors gleichzeitig belichtet werden, sind schnelle Messungen zeitgleich über eine große Fläche hinweg möglich. Der Flächensensor ist bevorzugt im wesentlichen parallel zur Oberfläche des Biochips orientiert, kann aber ansonsten weitgehend beliebig im Detektionssystem angeordnet sein, beispielsweise horizontal, vertikal oder in einer "upside down"-Orientierung.

Der direkte Kontakt bzw. der sehr geringe Abstand zwischen Flächensensor und Oberfläche des Biochips entspricht einer Art Kontaktbelichtung, wie sie von Filmen oder Photoplatten her bekannt ist, ohne allerdings deren spezifischen Nachteile in Kauf nehmen zu müssen: So ist herkömmlicherweise für jedes Bild ein neuer Film oder eine neue Photoplatte notwendig, die anschließend aufwendig entwickelt und fixiert werden müssen. Bevor die mit herkömmlichen Filmen oder Photoplatten aufgenommenen Bilder rechnerisch bearbeitet oder ausgewertet werden können, müssen sie nach der Entwicklung noch digitalisiert werden. Im Gegensatz dazu lassen sich die von dem bildgebenden photoelektrischen Flächensensor gelieferten Signale während der Belichtung digitalisieren und rechnerisch bearbeiten. Die Signalintegrationszeit ist variabel und kann je nach Art des verwendeten Bildsensors bzw. der Stärke des Chemilumineszenzsignals gewählt und gegebenenfalls sogar erst während der laufenden Messung endgültig festgelegt werden. Außerdem kann der Flächensensor oder das gesamte Detektionssystem, in das er integriert, ist nach einer Messung gewaschen, getrocknet und erneut benutzt werden

Wenn man einen Bildsensor, beispielsweise ein Video-CCD-Sensor mit niedrigem dynamischen Bereich verwendet, kann der Meßbereich durch geeignete Steuersoftware durch automatische Variation der Belichtungszeit auf mindestens 10 Bit ausgeweitet werden. Scientific-CCDs sind sogar mit einem Meßbereich von 12 bis 18 Bit erhältlich. Bei Verwendung von sogenannten "locally adaptive TFA-Sensoren" ist es sogar möglich, die von herkömmlicher CMOS- bzw. CCD-Technologien her bekannte dynamische Bandbreite von 70 dB durch Trennen der Pixelinformation in zwei separate Signale auf eine dynamische Bandbreite von 150 dB oder mehr zu erhöhen.

Das bei der Kontaktabbildung der Oberfläche eines Biochips erzielbare räumliche Auflösungsvermögen wird einerseits durch die Pixelgröße des Flächensensors und andererseits durch den Abstand des Biochips vom Flächensensor bestimmt. Sofern zwischen Flächensensor und Biochip ein Reaktionsraum zur Durchführung von Chemi- oder Biolumineszenzreaktionen vorgesehen ist, wird ein räumliches Auflösungsvermögen von 20 µm oder besser angestrebt. In Fällen, in denen ein direkter Kontakt von Flächensensor und Biochip realisiert werden kann, entspricht das Auflösungsvermögen der Pixelgröße des Sensor selbst.

Als Biochip können alle Formate gewählt werden, die über eine planare Oberfläche verfügen oder bei denen die aktiven Substanz nicht in Vertiefungen immobilisiert sind, die tiefer sind als die gewünschte räumliche Auflösung. Die Biochips weisen an einer planaren Trägeroberfläche immobilisierte Substanzen auf, wobei diese immobilisierten Substanzen an der Oberfläche fixierte biologische Sonden und/oder an die Sonden gebundene Proben sein können. Dabei können die Sonden, die Proben, die Sonden und die Proben oder gegebenenfalls weitere an die Sonden oder die Proben bindende Substratmoleküle markiert sein.

Als Trägermaterial können folgende Stoffe verwendet werden: Glas (Standardglas, Pyrexglas, Quarzglas), Kunststoffe bevorzugt hoher Reinheit bzw. geringer Eigenfluoreszenz (wie Polyolefine, z.B. PE (Polyethylen), PP (Polypropylen), Polymethylpenten, Polystyrol, PMMA (Poly(methylmethacrylat)), Polycarbonat, Teflon), Metalle (wie Gold, Chrom, Kupfer, Titan, Silizium), oxidische Materialien bzw. Beschichtungen (Keramiken, aluminiumdotiertes Zinkoxid (TCO), Silica, Aluminiumoxid). Die Trägermaterialien können als Membranen (wie Polysacharide, Polycarbonat, Nafion), dreidimensionale Strukturen (etwa Gele z.B. Polyacrylamid, Agarose, Keramiken) oder auch Formteile aus obigen Materialien, wie Folien und Dipsticks, ausgebildet sein. Für eine bessere Haftung, die Reduzierung unspezifischer Bindung oder für eine kovalente Ankopplung der Sonden kann das Aufbringen einer Zwischenschicht oder eine Voraktivierung der Oberfläche notwendig sein, z.B. durch Silane (Alkyisilane, Epoxysilane, Aminosilane, Carboxysilane), Polymere (Polysaccharide, Polyethylenglycol, Polystyrol, polyfluorierte Kohlenwasserstoffe, Polyolefine, Polypeptide), Alkylthiole, derivatisierte Alkylthiole, Lipide, Lipid-Doppelschichten oder Langmuir-Blodgett Membranen.

Die Aufbringung der Sonden auf die Oberfläche erfolgt durch Pipettieren, Dispensieren, Drucken, Stempeln oder in situ Synthese (wie z.B. photolithographische Techniken). Es werden bevorzugt verschiedene Sonden in einem zweidimensionalen Muster auf die Oberfläche aufgebracht. Jeder Sonde kann dann eine eindeutige Position auf der Oberfläche zugeordnet werden. Die Ankopplung der Sonden kann kovalent, adsorptiv oder über physikalisch/chemische Wechselwirkungen der Sonden mit der Oberfläche erfolgen. Es können alle bekannten Techniken eingesetzt werden.

Mit Sonden sind Strukturen gemeint, die mit einem oder mehreren Targets (Proben) in spezifische Wechselwirkung treten können. So dienen Biochip-Sonden in der Regel zur Untersuchung biologischer Targets, insbesondere Nukleinsäuren, Proteine, Kohlenhydrate, Lipide und Metabolite. Als Sonden werden bevorzugt verwendet: Nukleinsäuren und Oligonukleotide (einzel- und/oder doppelsträngige DNA, RNA, PNA, LNA in Reinform oder auch in Kombinationen), Antikörper (humane, tierische, polyklonale, monoklonale, rekombinante, Antikörperfragmente, z.B. Fab, Fab', F(ab)₂, synthetische), Proteine (etwa Allergene, inhibitoren, Rezeptoren), Enzyme (etwa Peroxidasen, Alkalische-Phosphatasen, Glukose-Oxidase, Nukleasen), kleine Moleküle (Haptene): Pestizide, Hormone, Antibiotika, Pharmaka, Farbstoffe, synthetische Rezeptoren oder Rezeptorliganden. Besonders bevorzugt handelt es sich bei den Sonden um Nukleinsäuren, insbesondere Oligonukleotide.

Gegenstand der vorliegenden Erfindung ist außerdem ein Verfahren zur ortsaufgelösten Detektion von elektromagnetischer Strahlung, insbesondere von Chemilumineszenz-, Biolumineszenz- und Fluoreszenzstrahlung, die von auf einer planaren Oberfläche eines Trägers immobilisierten Substanzen emittiert wird, wobei man einen bildgebenden photoelektrischen Flächensensor in geringem Abstand von der Oberfläche des Trägers anordnet, die immobilisierten Substanzen zur Emission von elektromagnetischer Strahlung anregt und die emittierte Strahlung ohne Verwendung eines abbildenden optischen Systems photoelektrisch detektiert.

Bevorzugt detektiert man eine von den immobilisierten Substanzen emittierte Chemilumineszenz- und Biolumineszenzstrahlung. In diesem Fall ist keine Einstrahlung von Anregungslicht erforderlich, so daß das erfindungsgemäße Detektionssystem besonders kostengünstig realisiert werden kann. Die Detektion von Lumineszenzstrahlung hat außerdem den Vorteil, daß die Strahlung direkt von der Oberfläche des planaren Trägers stammt und keine störende Streustrahlung aus dem den Träger bedeckenden Reaktionsraum oder dem Träger selbst ausgestrahlt wird.

Bei Chemi- oder Biolumineszenzmessungen kann das System so ausgelegt sein, daß schon die Bindung der Probe an die Sonde zur Lichtemission führt. In diesen Fällen werden die für die Lumineszenzreaktion erforderlichen Systemkomponenten durch Bildung eines Sonden/Proben-Komplexes bereitgestellt. Es ist auch möglich, daß erst nach der Bindung von Sonde und Probe in einem weiteren Schritt noch erforderliche Komponenten, z.B. ein geeignetes Chemilumineszenzsubstrat, zugegeben werden, die durch - nunmehr selbst an die fixierten Sonden gebundene - Proben zu lichtemittierenden Produkten umgesetzt werden. Vorzugsweise erzeugt man die Chemilumineszenzstrahlung auf der Oberfläche des planaren Trägers durch enzymatische Reaktionen. Dazu wird entweder ein Chemilumineszenzsubstrat oder ein Enzymkomplex auf dem Träger fixiert und eine Lösung des Enzyms bzw. eines Chemilumineszenzsubstrates zugegeben. Beim Umsatz des Substrates wird Licht emittiert. Jedenfalls werden die nachzuweisenden auf dem Biochip immobilisierten Substanzen üblicherweise mit einem Lumineszenzmarker versehen. Dies kann direkt erfolgen (Verwendung von Enzymen z.B. Meerrettichperoxidase (POD) oder Enzymsubstraten (z.B. Luminol)) oder über einen mehrstufigen Prozeß (Einführung eines primären Labels wie Biotin oder Digoxigenin (DIG) und Nachinkubation mit Lumineszenzmarkern wie PODmarkiertem Streptavidin oder anti-DIG). Der letzte Schritt ist üblicherweise die Zugabe von Enzymsubstratlösung oder, wenn Substratmoleküle wie Luminol als Marker verwendet wurden, von Enzymlösung. Die Verwendung von Enzymen als Marker hat den Vorteil, daß durch die Enzymreaktion eine enorme Signalamplifikation erreicht wird. Alle chemilumineszierenden oder biolumineszierenden Systeme sind als Marker zur Signalerzeugung bei der Biochipauswertung denkbar, z.B. Alkalische Phosphatase mit Dioxetan- (AMPPD) oder Acridiniumphosphat-Substraten; Meerrettichperoxidase mit Luminol- oder Acridiniumester-Substraten; Mikroperoxidasen oder Metallporphyrinsysteme mit Luminot; Glucoseoxidase, Glucose-6-phosphatdehydrogenase; oder auch Luciferin/Luciferase-Systeme.

Gemäß einer anderen Ausführungsform der Erfindung wird eine von den immobilisierten Substanzen emittierte Fluoreszenzstrahlung detektiert. Gegenüber den enzymatischen Chemilumineszenzsystemen haben Fluoreszenzfarbstoffe den Vorteil, daß die Messung direkt nach Einführung des Markers erfolgen kann. Enzym- oder Proteinmarker (beispielsweise der häufig eingesetzte Biotin/Streptavedin-Komplex) benötigen dagegen einen weiteren Inkubationsschritt, in welchem der Enzymmarker eingeführt und die Substratlösung zugegeben wird. Allerdings ist für Fluoreszenzmessungen die Einstrahlung von Anregungslicht notwendig. Da der Flächensensor und die Oberfläche des Biochips nur in einem geringen Abstand voneinander angeordnet sind, wird vorzugsweise ein Träger verwendet, in den beispielsweise von der dem Träger abgewandten Rückseite her Anregungslicht eingekoppelt werden kann. Das eingekoppelte Anregungslicht wird dann unter Totalreflexion in dem Träger geführt, wobei die Anregung der auf der Oberfläche des Trägers immobilisierten Substanzen durch evaneszentes Licht erfolgt. Hierbei ist selbstverständlich auf ein möglichst fluoreszenzarmes Trägermaterial zu achten. Zur Unterdrückung von Streulichtanteilen in dem vom Flächensensor detektierten Signal können Sensoren mit schneller Ansprechzeit verwendet werden. Bei Anregung mit kurzen Lichtimpulsen kann dann der Streulichtanteil durch zeitliche Diskriminierung vom eigentlich interessierenden, zeitlich verzögerten Fluoreszenzsignal unterschieden werden.

Es ist erfindungsgemäß schließlich auch möglich, die zu untersuchenden Substanzen direkt auf dem photoelektrischen Flächensensor zu immobilisieren. In diesem Fall dient der Flächensensor gleichzeitig als Träger für die Substanzen. Der Träger kann dann beispielsweise eine dünne Quarzschicht sein, die direkt auf den photoelektrischen Zellen des Flächensensors als Schutzschicht vorgesehen ist. Die Oberfläche des Flächensensors kann dazu auch geeignet beschichtet sein (z.B. kann eine hydrophobe Oberfläche durch Silanisierung erzeugt werden).

Der Flächensensor kann in eine Durchflußzelle integriert werden. Es können zusätzliche Einrichtungen zur Substratzugabe, zum Waschen oder zum Trocknen vorgesehen sein.

Die vorliegende Erfindung kann beispielsweise zur Auswertung von nichtkompetitiven oder kompetitiven Testverfahren genutzt werden. Bei nichtkompetitiven bindet die zu analysierende Probe an die auf der Oberfläche des Biochips vorab immobilisierte Sonde. Die Probe kann man zuvor mit einem Chemilumineszenzmarker versehen. Es ist auch möglich, daß die Probe zuerst an die fixierte Sonde bindet und in einem zweiten Schritt markiert wird (z.B. bei Primer Extension oder Rolling Cycle PCR). In all diesen Fällen erhält man ein Meßsignal, das um so größer ist, je mehr gebundene Probenmoleküle vorhanden ist. Es ist auch möglich, daß durch die Wechselwirkung der Probe mit den auf der Oberfläche immobilisierten Sonden die Aktivität des die Chemilumineszenz katalysierenden Enzyms verändert wird (Abschwächung, Verstärkung, z.B. Enzymhemmtests) und diese Änderung als Meßsignal registriert wird. Als Beispiele für nichtkompetitive Testverfahren können Hybridisierungsreaktionen von PCR-Produkten oder markierter DNA/RNA an auf der Oberfläche immobilisierte Oligonukleotide oder cDNA oder Sandwichimmunoassays genannt werden. Bei kompetitive Testverfahren wird der Probe eine markierte Substanz zugesetzt, die ähnliche Bindungseigenschaften zu den auf der Oberfläche immobilisierten Sonde hat wie die Probe selbst. Es findet eine Konkurrenzreaktion zwischen Probe und Marker um die begrenzte Anzahl von Bindungsplätzen auf der Oberfläche statt. Man erhält ein Signal, das um so niedriger ist, je mehr Probenmoleküle vorhanden ist. Beispiele hierfür sind Immunoassays (ELISA) oder Rezeptorassays.

Die vorliegende Erfindung wird im folgenden unter Bezugnahme auf in den beigefügten Zeichnungen dargestellte Ausführungsbeispiele ausführlicher beschrieben.

In den Zeichnungen zeigt:
- Figur 1: eine schematische Explosionsdarstellung einer Vorrichtung zur Kontaktabbildung der von einem Biochip emittierten Chemilumineszenzstrahlung;
- Figur 2: einen Teilschnitt der Anordnung von Flächensensor und Biochip der Vorrichtung der Figur 1;
- Figur 3:: einen Teilschnitt einer alternativen Anordnung von Flächensensor und Biochip zur Detektion von Chemilumineszenzstrahlung;
- Figur 4:: eine alternative Anordnung von Biochip und Flächensensor zur Detektion von Fluoreszenzstrahlung;
- Figur 5a:: eine CCD-Kontaktbelichtungsaufnahme der von einem Biochips emittierten Chemilumineszenzstrahlung;
- Figur 5b:: den Intensitätsverlauf des Chemilumineszenzsignals entlang einer Linie in Figur 5a;
- Figur 6a: eine mit einem Röntgenfilm gewonnene Aufnahme des Biochips der Figur 5a;
- Figur 6b: den Intensitätsverlauf entlang einer Linie in Figur 6a;
- Figur 7:: eine CCD-Kontaktbelichtungsaufnahme der von einem Protein-Chip emittierten Chemilumineszenzstrahlung;
- Figur 8:: eine CCD-Kontaktbetichtungsaufnahme der von einem DNA-Chip emittierten Chemilumineszenzstrahlung;
- Figur 9:: ein Diagramm, das die Intensität des Chemilumineszenzsignals in Abhängigkeit von der immobilisierten Oligonukleotidkonzentration wiedergibt;
- Figur 10:: ein Diagramm, das zeigt, wie bei dem erfindungsgemäßen Verfahren der Meßbereich durch unterschiedliche Belichtungszeiten vergrößert werden kann;
- Figur 11:: eine CCD-Kontaktbelichtungsaufnahme der von einem weiteren DNA-Chip emittierten Chemilumineszenzstrahlung;
- Figur 12:: eine Diagramm, das eine aus der Aufnahme der Figur 11 ermittelte Diskriminierungsrate veranschaulicht;
- Figur 13:: eine CCD-Kontaktbelichtungsaufnahme eines diagnostischer Biochips zur Mutationsbestimmung in Onkogenen.

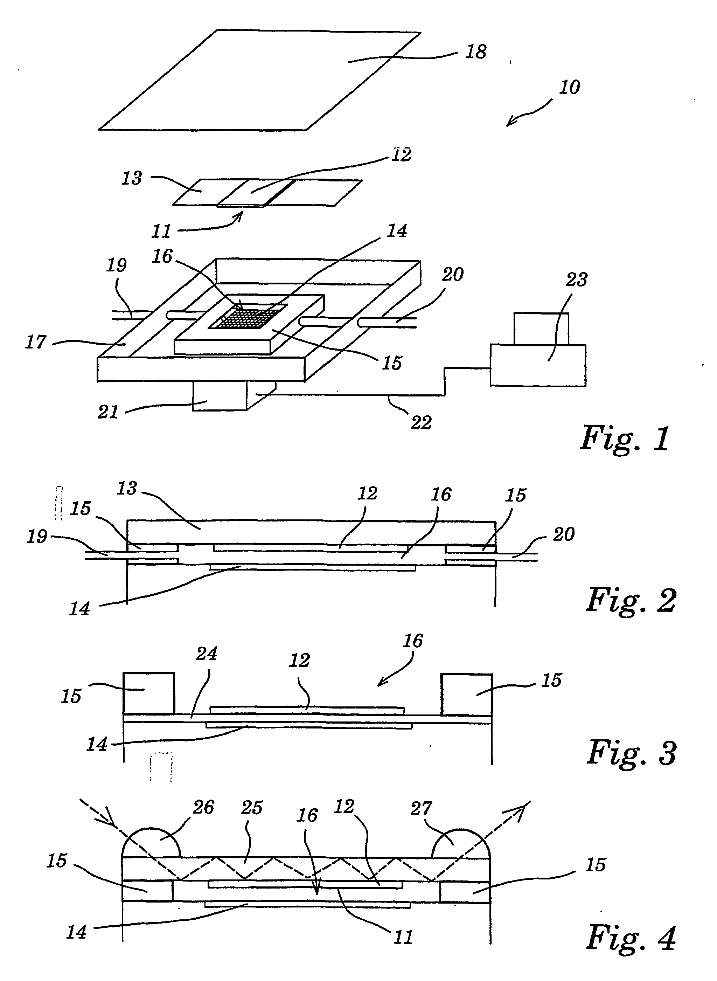

In Figur 1 ist schematisch eine Explosionsdarstellung eines Detektionssystems 10 zur Durchführung des erfindungsgemäßen Verfahrens dargestellt. Im dargestellten Beispiel dient das Detektionssystem 10 zur ortsaufgelösten Messung von Chemilumineszenzstrahlung. Die Chemilumineszenzstrahlung wird von Substanzen emittiert, die auf der planaren Oberfläche 11 eines funktionalisierten Bereichs 12 eines Biochips 13 immobilisiert sind. Dazu wird der Biochip 13 in möglichst geringem Abstand von einem Flächensensor 14, beispielsweise einem CCD-Chip, angeordnet (vergl. Fig. 2). Der Flächensensor 14 kann dann ohne Zwischenschaltung eines abbildenden optischen Systems, wie beispielsweise einer Linsen- oder einer Faseroptik, ein ortsaufgelöstes zweidimensionales Abbild der von der Oberfläche des funktionalisierten Bereichs 12 emittierten Chemilumineszenzstrahlung detektieren. In dem in Figur 1 dargestellten Beispiel liegt der Biochip 13 auf einer den Flächensensor 14 umgebende Dichtung 15. Die Dichtung 15 besitzt eine Höhe, die so gewählt ist, daß bei aufgelegtem Biochip zwischen der Oberfläche 11 des funktionalisierten Bereichs 12 und dem Flächensensor 14 ein Reaktionsraum 16 ausgespart bleibt, der beispielsweise vor Auflegen des Biochips 13 mit einer in der Regel wäßrigen Lösung eines Chemifumineszenzsubstrates gefüllt werden kann. Die gesamte Anordnung aus Biochip und Flächensensor ist von einem Gehäuse 17 umgeben, welches durch einen Deckel 18 lichtdicht verschlossen werden kann. Nach Auflegen des Biochips 13 wird Chemilumineszenzsubstrat von im funktionalisierten Bereich 12 immobilisierten Enzymen umgesetzt, was mit einer Lichtemission verbunden ist. Der Abstand zwischen der Oberfläche des Biochips 13 und dem Flächensensor 14 ist so gewählt, daß jedes Pixelelement des Sensors 14 im wesentlichen nur Licht aus unmittelbar gegenüberliegenden Bereichen des Biochips empfängt. Der Abstand zwischen Flächensensor und Biochip sollte daher nicht wesentlich größer als die Kantenlänge eines Pixels des Flächensensors 14 sein. Typischerweise liegt dieser Abstand dann im Bereich von 5 -100 µm. Als Obergrenze des Abstandes ist jedenfalls der Durchmesser der einzelnen Feldelemente auf dem Biochip selbst anzusehen, da diese Feldelemente noch eindeutig voneinander unterschieden werden müssen.

Bei einer besonders einfache Ausführungsform der Vorrichtung zur Durchführung des erfindungsgemäßen Verfahrens kann die Substratlösung manuell in den Reaktionsraum 16 gefüllt werden. Bei einer automatisierten Anordnung können dazu beispielsweise auch Pipettierroboter verwendet werden. Es ist jedoch auch möglich, den Reaktionsraum 16 mit Hilfe von einer oder mehreren Leitungen 19, 20 zu befüllen bzw. zu spülen. Prinzipiell können folgende Automatisierungsschritte einzeln oder in Kombination realisiert werden: Einlegen bzw. Wechseln der Biochips 13, Zugabe von Substratlösung, sowie nach der Messung Waschen und Trocknen des Sensors 14. Der Flächensensor kann in eine Flußzelle, insbesondere in ein automatisches oder manuelles Fließinjektionssystem (FIA-System) als Teil einer Durchflußzelle, integriert sein. Dabei können Flächensensor und Biochip mittels Abstandshalter die Flußzelle definieren.

Das von dem Flächensensor 14 detektierte Chemilumineszenzlicht der einzelnen Pixetetemente wird mittels einer Steuerelektronik 21 digitalisiert und über eine Datenleitung 22 zu einem Computer 23 übertragen, der auch die Steuerung der Bildaufzeichnung, die Bildverarbeitung und Speicherung der Daten übernimmt. In einer speziellen Ausführungsform ist der Computer direkt in das System integriert.

In Figur 2 ist die Anordnung der Figur 1 im Teilschnitt in einem größeren Maßstab dargestellt. Die dargestellten Elemente sind mit denselben Bezugsziffem wie in Figur 1 bezeichnet.

Figur 3 zeigt eine Variante einer Meßanordnung zur Detektion von Chemilumineszenzstrahlung, bei welcher der Biochip aus einer dünnen Folie 24 besteht, die direkt auf dem Flächensensor 14 liegt. Die Folie 24 weist beispielsweise nur eine Dicke von 10 µm auf und ist für das Chemilumineszenzlicht durchlässig. Diese Variante besitzt den Vorteil, daß die Dicke des Reaktionsraums 16 beliebig gewählt werden kann, da dieser sich auf der vom Sensor 14 abgewandten Seite der Folie 24 befindet und daher keinen Einfluß auf das Auflösungsvermögen des Detektionssystems hat.

In Figur 4 ist eine Meßanordnung zur Detektion von Fluoreszenzlicht dargestellt. Der funktionalisierte Bereich 12 befindet sich auf einem für Anregungslicht transparenten Biochip 25.

Beispielsweise mittels zweier an gegenüberliegenden Seitenkanten des Trägers aufgeklebter Prismen 26, 27 wird Anregungslicht (in Fig. 4 als gestrichelte Linie angedeutet) in den Biochip 25 eingekoppelt. Ein evaneszenter Anteil des Anregungslichtes regt die auf der Oberfläche 11 des funktionalisierten Bereichs 12 fixierten fluoreszenzmarkierten Substanzen zur Emission von Fluoreszenzlicht an, das dann von dem Flächensensor 14 registriert wird. Besteht das Anregungslicht und folglich auch das emittierte Fluoreszenzlicht aus kurzen Lichtimpulsen, kann eine nachgeschaltete Elektronik das vom Flächensensor 14 registrierte Signal in einen möglicherweise vorhandenen Streulichtanteil und einen eigentlich interessierenden, zeitlich etwas verzögert emittierten Fluoreszenzanteil trennen. Eine zwischen Biochip und Flächensensor angeordnete Filtereinrichtung zur Ausblendung des Streulichtanteils ist daher nicht erforderlich.

### Beispiele

Die folgenden Beispiele wurden mit einem einfachen Aufbau für eine manuelle Bedienung, wie er in Figur 1 schematisch dargestellt ist, durchgeführt. Es wird ein Interline-Flächensensor mit Videofrequenz verwendet (Sony, 768 x 576 Pixel). Derartige CCD-Sensoren sind kommerziell nur gekapselt erhältlich, d.h. der Sensor ist in ein Gehäuse aus Keramik integriert und nach oben mit einer transparenten Abdeckung aus Glas verschlossen. Der Flächensensor wurde durch Entfernen der Abdeckung freigelegt, so daß ein für eine scharfe Abbildung erforderlicher geringer Abstand zwischen Sensor und Biochip realisiert werden kann. Nach Freilegen des Sensors wurde die Elektronik durch Abdichten mit einer Vergußmasse geschützt, um Kurzschlüsse bei der Zugabe der wäßrigen Substratlösung zu verhindern.

Zugabe der Substratlösung, Einlegen des Biochips, sowie nach der Messung Waschen mit Wasser und Trocknen des CCD-Sensors wurden von Hand durchgeführt. Zum Schutz des CCD-Sensors kann auf diesem eine dünne Folie (z.B. mit einer Dicke von 3 µm) oder eine dünne Schutzschicht (z.B. Lackschicht) aufgebracht werden. Die auf einem CCD-Chip normalerweise vorhandene dünne SiO₂-Schicht reicht aber zum Schutz vor der wäßrigen Substratlösung aus.

Die Ausieseeiektronik ist in einem Kameramodul untergebracht. Das Videosignal wird von einem 8 Bit Frame-Grabber eines Computers digitalisiert Durch die Steuerung der On-Chip Integration wird eine beträchtliche Empfindlichkeitssteigerung erreicht. Außerdem läßt sich durch unterschiedliche Belichtungszeiten der dynamische Bereich des Systems erweitern. Die digitalisierten Bilddaten lassen sich direkt in einem gängigen Grafikformat abspeichem und stehen für eine weitere Bearbeitung unmittelbar zur Verfügung.

Steuerung und Bilderfassung kann direkt in einem Speicherchip des Detektionssystems oder extern über einen PC oder Laptop erfolgen.

### Beispiel 1: Optischen Auflösung

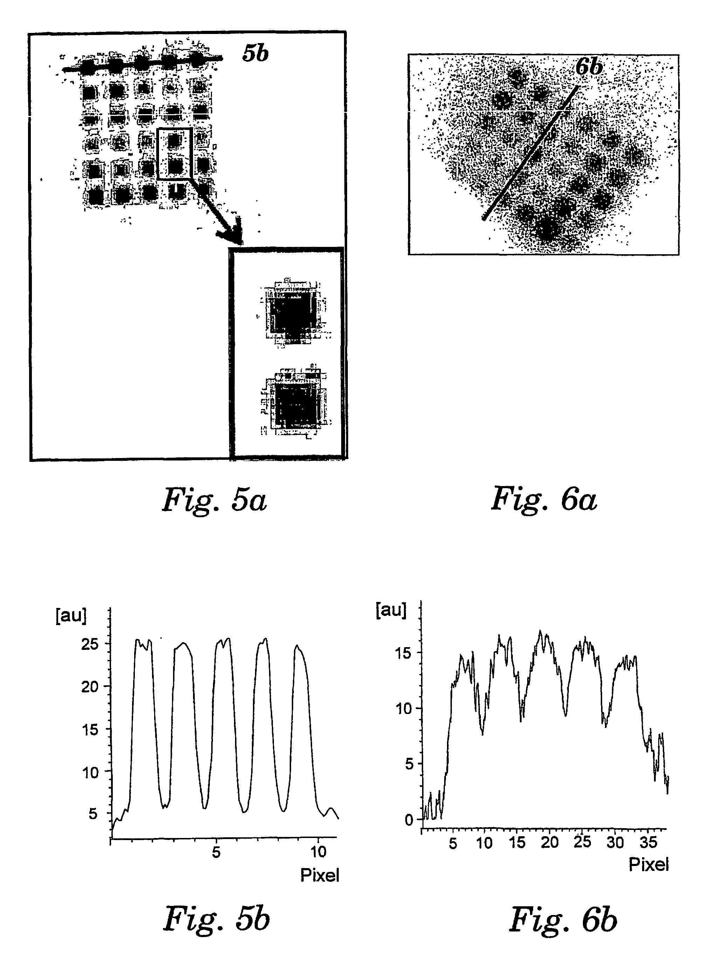

Figur 5a zeigt als Beispiel für das räumliches Auflösungsvermögen des CCD-Sensors die Aufnahme eines Biochips. Als Träger dienten Glasoberflächen, auf die ein Array mit 5x6 Feldelementen aus einer dünnen Goldschicht sehr präzise aufgebracht wurde. Es handelte sich um mikrostrukturierte quadratische Goldoberflächen mit einer Seitenlänge von 100 µm und einem Mittenabstand (Raster) von 200 µm. Die Goldoberflächen wurden durch Behandlung mit einer HPDP-Biotinlösung (Pierce) biotinyliert. Um die gesamte Oberfläche mit SH-Gruppen abzusättigen, wurden die Chips in einem zweiten Schritt mit Mercaptohexanol behandelt. An das immobilisierte Biotin konnte nun ein mit Meerrettichperoxidase (POD) markiertes Streptavidin (Streptavidin-POD, Sigma, Stammlösung 1 mg/mL; Verdünnung 1:10.000) binden. Nach einem Waschschritt und Inkubation mit Chemilumineszenzsubstrat (SuperSignal Femto, ELISA, Chemilumineszenzsubstrat, Pierce) wurde der Chip vermessen. Bei der Aufnahme der Figur 5a wurde eine Belichtungszeit von 12 s eingestellt. Ein Teilbild der Figur 5a zeigt zwei Feldelemente in vergrößerter Darstellung, so daß bereits die einzelnen Pixel des CCD-Sensors sichtbar werden. In Figur 5b ist der Verlauf des Chemilumineszenzsignals entlang der Linie "5b" der Figur 5a dargestellt. Für Vergleichsmessungen wurde der Biochip mit einem Röntgenfilm (Medical X-Ray Film, Fuji RX, No. 036010) durch Kontaktbelichtung vermessen. In Abbildung 6a ist das mit dem Röntgenfilm bei einer Belichtungszeit von ebenfalls 12s erzielte Ergebnis dargestellt. Figur 6b zeigt die Schwärzungskurve des Films entlang der Linie "6b" der Figur 6a. Insbesondere aus dem Vergleich der Figuren 5b und 6b wird deutlich, daß mit dem erfindungsgemäßen Meßaufbau Setup 100 µm Strukturen gut aufgelöst werden können und die erhaltene Auflösung, sowie das Signal/Rausch-Verhältnis besser sind als mit dem verwendeten Standardröntgenfilm. Die Empfindlichkeit des CCD-Chips entsprach in diesem Format der des Röntgenfilms.

### Beispiel 2: Protein-Chip

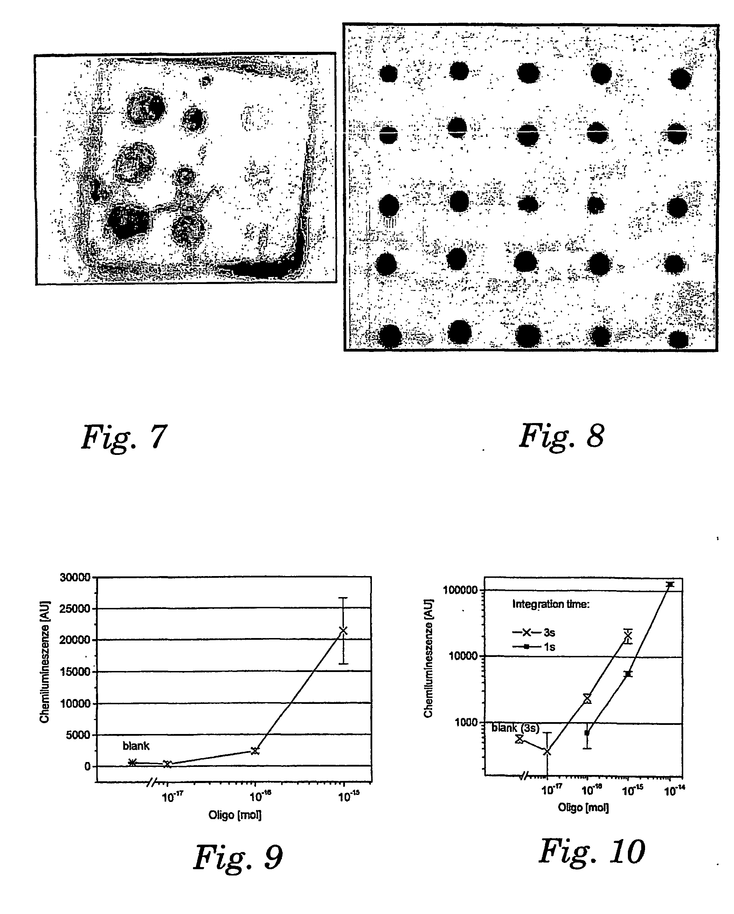

Die Oberfläche eines Glasobjektträgers wurde mit Trimethylchlorsilan silanisiert. Auf dieser Oberfläche wurde ein Anti-Peroxidaseantikörper (Anti-Peroxidaseantikörper, rabbit, Sigma) in einzelnen Spots und unterschiedlichen Konzentrationen adsorptiv immobilisiert. Nach einer Inkubationszeit von 3 h wurde die Oberfläche mit einer Mischung aus BSA und Casein qeblockt. Die Chips wurden nun 30 - 60 min mit unterschiedlichen Konzentrationen an Peroxidase (Peroxidase aus Meerrettich, Grade I, Boehringer, Stammlösung 5 mg/mL) inkubiert, gewaschen, mit Chemilumineszenzsubstrat versetzt und mit dem Detektionssystem (CCD-Chip, 3 µm Folie, 1,5 µL Substratlösung, Biochip aufgelegt) vermessen. Das Ergebnis der Messung ist in Figur 7 dargestellt. Man erkennt einen Protein-Chip mit manuell aufgebrachten Spots mit 3 verschiedenen Anti-POD-Antikörperkonzentrationen (Spalten von links nach rechts: Verdünung 1:100, 1:1.000, 1:10.000; POD-Verdünnung 1:10⁶; Belichtungszeit 35 s). Bei diesem Testformat war die Empfindlichkeit um eine Größenordnung höher als unter identischen Bedingungen mit dem Röntgenfilm.

### Beispiel 3: DNA-Chip

Auf Kunststoffträgem wurden 18-mer Oligonukleotide kovalent immobilisiert (Sequenz: 5'TATTCAGGCTGGGGGCTG-3'). Es wurde mit einer komplementären 18-mer Sonde, die am 5'-Ende biotinyliert war, hybridisiert (5xSSP, 0.1% Tween, 1h). Nach einem Waschritt wurde mit Streptavidin-POD inkubiert (Verdünnung 1:100; 5xSSP, 0.1% Tween 20; 30 min) und nach Waschen mit dem bereits im Beispiel 2 beschriebenen Aufbau vermessen. Figur 8 zeigt eine Übersicht eines homogen in einem Array aus 5x5 Feldelementen gespotteten Biochips. Es ist auch möglich das Streptavidin-POD bereits der Hybridisierungslösung zuzusetzen. Auf diese Weise wird die Zahl der Inkubations- und Waschschritte reduziert und der Test ist in Durchführbarkeit und Schnelligkeit (bis auf die Substratzugabe) mit Fluoreszenzsystemen vergleichbar.

Die Nachweisgrenze für die Detektion von DNA wurde bestimmt, indem DNA-Chips nach oben beschriebenem Schema mit ansteigenden Konzentrationen an Biotinsonde hybridisiert wurden. Das Ergebnis ist in Figur 9 dargestellt. Die Nachweisgrenze liegt in diesem Format (Belichtungszeit 1s) bei einer Absolutmenge an DNA von unter 10⁻¹⁶ mol und wird durch den Hintergrund, d.h. die unspezifische Bindung von Streptavidin-POD an die immobilisierten Oligonukleotide limitiert.

Der dynamische Bereich des hier verwendeten Video-CCD-Chips von 8 Bit kann durch unterschiedliche Belichtungszeiten ausgeglichen werden. Die Ergebnisse entsprechender experimenteller Untersuchungen sind im Diagram der Fig. 10 dargestellt. Auf diese Weise ist eine Erweiterung des Meßbereiches auf 10 Bit und darüber möglich.

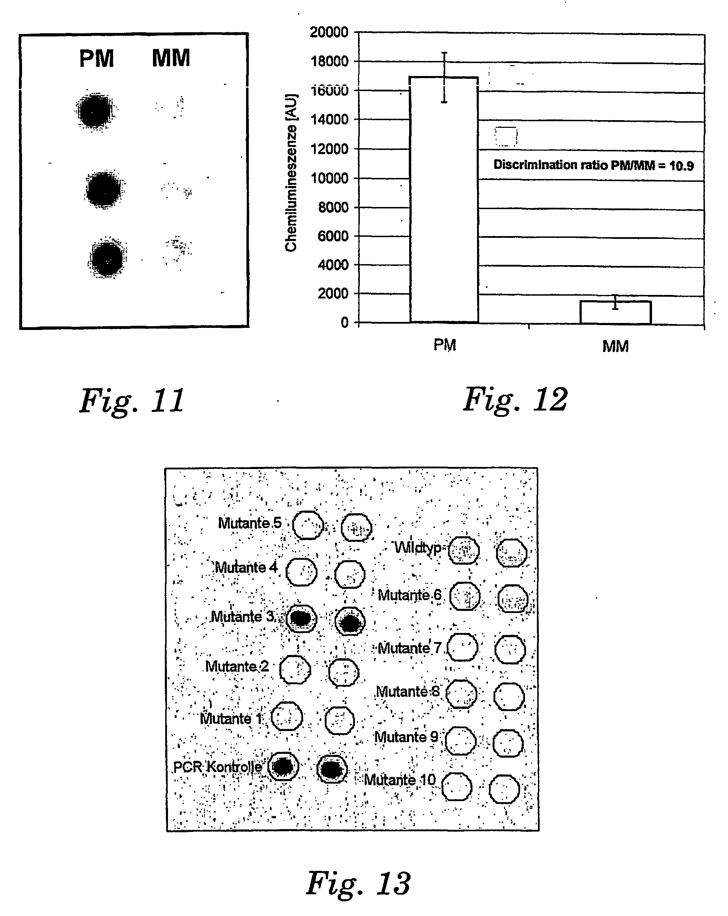

Wie bei anderen Detektionsmethoden ist durch die Wahl von stringenten Bedingungen (20 min Waschen mit 0.3xSSPE und 25% Formamid nach der Hybridisierung) eine gute Diskriminierung zwischen Perfect-Match (PM) und einfachen Mismatch (MM) Proben möglich. Für die oben erwähnten immobilisierten 18-mer Oligonukleotide werden bei Einführung eines CC-Mismaches an Position 7 Diskriminierungsraten von über 10 erreicht. In Figur 11 ist die entsprechende CCD-Kontaktbelichtungsaufnahme des resultierenden Chemilumineszenzsignals eines DNA-Chips dargestellt (Spots der linken Spalte: Perfect-Match (PM); Spots der rechten Spalte Mismatch (MM). Die Signalintensitäten sind im Diagramm der Figur 12 dargestellt. Das lntensitätsverhältnis von PM/MM beträgt im dargestellten Beispiel 10,9.

### Beispiel 4: Diagnostischer Biochip - Mutationsbestimmung in Onkogenen

Es wurde ein DNA-Chip mit verschiedenen 13mer Capture-Probes (immobilisierte Sonden) für 10 Mutationen eines Onkogens hergestellt Zusätzlich wurde eine Sonde für den Wildtyp und eine PCR-Kontrolle integriert. Aus einer Zellinie wurde DNA mit der Mutation 3 (siehe Tabelle unten) isoliert und mittels mutationsanreichender PCR amplifiziert (50 µL Ansatz mit ca. 10 ng DNA; 35 Zyklen; Primer an 5'-Ende biotinyliert; Amplifikationslänge ca. 157 Basenpaare). Das PCR-Produkt wurde mit 20xSSPE auf 6xSSPE eingestellt und mit 6xSSPE 1:10 verdünnt. Vor der Hybridisierung wurde 1:1 mit einer 1:100-Verdünnung von Streptavidin-POD in 6xSSPE versetzt. Nach der Hybridisierung wurde mit 6xSSPE gewaschen, der Chip mit Chemilumineszenzsubstrat versetzt und im Detektor vermessen. Die Zuordnung der jeweiligen Mutante (Diskriminierungsraten von über 20 zu anderen Mutanten) ist eindeutig möglich. In Figur 13 ist das Ergebnis der entsprechende Chemilumineszenzmessung dargestellt (Biochip nach stringenter Hybridisierung (1h, 37°C 6xSSPE); 3s Belichtung).

Es wurden folgende Diskriminierungsraten gemessen:

| Capture Probe | Diskriminierung zur Mutante 3 |
|---|---|
| PCR-Kontrolle | 1.11 |
| Mutante 3 | 1.00 |
| Mutante 1 | 21.5 |
| Mutante 5 | 64.4 |
| Mutante 6 | 71.1 |
| Wildtyp | 10.5 |

Für alle anderen Mutationen liegt die Diskriminierung bei >70.

## Patentansprüche

1. Verwendung eines bildgebenden photoelektrischen Flächensensors zur Kontaktabbildung der von einer Oberfläche eines Biochips emittierten Chemi- oder Biolumineszenzstrahlung, wobei der Flächensensor in eine Messzelle integriert ist, die als Durchflusszelle ausgebildet ist.

2. Verwendung gemäß Anspruch 1, **dadurch gekennzeichnet, dass** der Flächensensor ein Diodenarray, ein CCD-Sensor oder ein TFA-Bildsensor ist.

3. Verfahren zur ortsaufgelösten Detektion von elektromagnetischer Strahlung, die von auf einer Oberfläche eines Trägers immobllisierten Substanzen emittiert wird, wobei man
einen bildgebenden photoelektrischen Flächensensor in geringem Abstand von der Oberfläche des Trägers anordnet, so dass zwischen dem Flächensensor und dem Träger ein Reaktionsraum einer Durchflusszelle definiert wird,
den Reaktionsraum mit einer Enzym- oder Substratlösung spült, um die immobilisierten Substanzen zur Emission von Chemi- oder Biolumineszenzstrahlung anzuregen, und
die emittierte Strahlung ohne Verwendung eines abbildenden optischen Systems, photoelektrisch detektiert.

4. Verfahren gemäß Anspruch 3, **dadurch gekennzeichnet, dass** man den Sensor nach der Messung wäscht und trocknet.

5. Verfahren gemäß Anspruch 4, **dadurch gekennzeichnet, dass** das Spülen, Messen, Waschen und Trocknen automatisiert ist.

6. Verfahren gemäß einem der Ansprüche 3 bis 4, **dadurch gekennzeichnet, daß** man von den immobilisierten Substanzen emittierte Chemilumineszenzstrahlung detektiert.

7. Verfahren gemäß Anspruch 6, **dadurch gekennzeichnet, daß** man die Chemilumineszenzstrahlung durch enzymatische Reaktionen erzeugt.

8. Verfahren gemäß einem der Ansprüche 3 bis 4, **dadurch gekennzeichnet. daß** man von den immobillsierten Substanzen emittierte Biolumineszenzstrahlung detektiert.

9. Verfahren zur ortsaufgelösten Detektion von elektromagnetischer Strahlung, die von auf einer Oberfläche eines Trägers immobilisierten Substanzen emittiert wird, wobei man
die Substanzen auf einem als Träger dienenden photoelektrischen Flächensensor immobilisiert,
den Flächensensor in einer als Durchflusszelle ausgebildeten Messzelle anordnet, so dass ein Reaktionsraum definiert wird,
den Reaktionsraum mit einer Enzym- oder Substratlösung spült, um die immobilisierten Substanzen zur Emission von Chemi- oder Biolumineszenzstrahlung anzuregen, und
die emittierte Strahlung ohne Verwendung eines abbildenden optischen Systems photoelektrisch detektiert.

## Claims

1. The use of an image-generating photoelectric area sensor for contact imaging of the chemiluminescence radiation emitted by a surface of a biochip, the area sensor being integrated into a measuring cell which is designed as a flow cell.

2. The use as claimed in claim 1, **characterized in that** the area sensor is a diode array, a CCD sensor or a TFA image sensor.

3. A method for the spatially resolved detection of electromagnetic radiation which is emitted by substances immobilized on a surface of a support, which method comprises
arranging an image-generating photoelectric area sensor at a short distance from the surface of the support, so that a reaction space of a flow cell is defined between the area sensor and the support,
flushing the reaction space with a solution of enzyme or substrate in order to excite the immobilized substances so that they emit chemiluminescence or bioluminescence radiation, and
detecting photoelectrically the emitted radiation without using an imaging optical system.

4. The method as claimed in claim 3, **characterized in that** the sensor is washed and dried after the measurement.

5. The method as claimed in claim 4, **characterized in that** flushing, measuring, washing and drying are automated.

6. The method as claimed in either of claims 3 and 4, **characterized in that** chemiluminescence radiation emitted by the immobilized substances is detected.

7. The method as claimed in claim 6, **characterized in that** said chemiluminescence radiation is generated by enzymic reactions.

8. The method as claimed in either of claims 3 and 4, **characterized in that** bioluminescence radiation emitted by the immobilized substances is detected.

9. A method for the spatially resolved detection of electromagnetic radiation which is emitted by substances immobilized on a surface of a support, which method comprises
immobilizing said substances on a photoelectric area sensor used as support,
arranging said area sensor in a measuring cell designed as flow cell so that a reaction space is defined,
flushing the reaction space with a solution of enzyme or substrate in order to excite the immobilized substances so that they emit chemiluminescence or bioluminescence radiation, and
detecting photoelectrically the emitted radiation without using an imaging optical system.

## Revendications

1. Utilisation d'un capteur surfacique photoélectrique générateur d'images pour la formation d'images de contact du rayonnement de bioluminescence ou de chimioluminescence émis par une surface d'une biopuce, dans laquelle le capteur surfacique est intégré dans une cellule de mesure conçue en tant que cellule de débit.

2. Utilisation selon la revendication 1, **caractérisée en ce que** le capteur surfacique est un réseau de diodes, un capteur CCD et un capteur d'images TFA.

3. Procédé de détection déclenchée localement d'un rayonnement électromagnétique qui est émis par des substances immobilisées sur une surface d'un support, dans lequel
on dispose un capteur surfacique photoélectrique générateur d'images à une faible distance de la surface du support de sorte qu'un espace de réaction d'une cellule de débit est défini entre le capteur surfacique et le support,
on balaie l'espace de réactioh avec une solution enzymatique ou par une solution de substrat afin d'exciter les substances immobilisées pour émettre un rayonnement de chimioluminescence ou de bioluminescence,
on détecte le rayonnement émis de manière photoélectrique sans utiliser de système optique de formation d'images.

4. Procédé selon la revendication 3, **caractérisé en ce que** le capteur est lavé et séché après la mesure.

5. Procédé selon la revendication 4, **caractérisé en ce que** le balayage, la mesure, le lavage et le séchage sont automatisés.

6. Procédé selon l'une quelconque des revendications 3 à 4, **caractérisé en ce qu'**on détecte le rayonnement de chimioluminescence émis par les substances immobilisées.

7. Procédé selon la revendication 6, **caractérisé en ce qu'**on produit le rayonnement de chimioluminescence par des réactions enzymatiques.

8. Procédé selon l'une quelconque des revendications 3 à 4, **caractérisé en ce qu'**on détecte le rayonnement de bioluminescence émis par les substances immobilisées.

9. Procédé de détection déclenchée localement d'un rayonnement électromagnétique qui est émis par des substances immobilisées sur une surface d'un support, dans lequel
on immobilise les substances sur un capteur surfacique photoélectrique servant de support,
on dispose le capteur surfacique dans une cellule de mesure conçue en tant que cellule de débit, de manière à définir un espace de réaction,
on balaie l'espace de réaction avec une solution enzymatique ou une solution de substrat, afin d'exciter les substances immobilisées pour l'émission d'un rayonnement de chimioluminescence ou de bioluminescence
et
on détecte le rayonnement émis de manière photoélectrique sans utiliser de système optique de formation d'images.
